(19) Europäisches Patentamt
European Patent Office
Office européen des brevets

(11) Veröffentlichungsnummer: **0 269 009 B1**

(12) # EUROPÄISCHE PATENTSCHRIFT

(45) Veröffentlichungstag der Patentschrift: **31.07.91**

(51) Int. Cl.$^5$: **C07D 311/72**, C07D 303/04, C07C 43/23

(21) Anmeldenummer: **87117127.8**

(22) Anmeldetag: **20.11.87**

(54) **Verfahren zur Herstellung von Carbinolderivaten.**

(30) Priorität: **28.11.86 CH 4772/86**
**29.09.87 CH 3809/87**

(43) Veröffentlichungstag der Anmeldung:
**01.06.88 Patentblatt 88/22**

(45) Bekanntmachung des Hinweises auf die Patenterteilung:
**31.07.91 Patentblatt 91/31**

(84) Benannte Vertragsstaaten:
**AT BE CH DE FR GB IT LI NL**

(56) Entgegenhaltungen:
**EP-A- 0 107 806**
**EP-A- 0 129 252**
**EP-A- 0 257 503**

(73) Patentinhaber: **F. HOFFMANN-LA ROCHE AG**
**Postfach 3255**
**CH-4002 Basel(CH)**

(72) Erfinder: **Barner, Richard, Dr.**
**Traubenweg 16**
**CH-4108 Witterswil(CH)**
Erfinder: **Hübscher, Josef**
**Säspelstrasse 1**
**CH-4208 Nunningen(CH)**

(74) Vertreter: **Urech, Peter, Dr. et al**
**Grenzacherstrasse 124 Postfach 3255**
**CH-4002 Basel(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 269 009 B1

## Beschreibung

Die vorliegende Erfindung betrifft ein neues Verfahren zur Herstellung von tert. Methylcarbinolderivaten, welche als Zwischenprodukte für die Herstellung von d-α-Tocopherol (natürliches Vitamin E) geeignet sind, sowie ein Verfahren zur Herstellung von d-α-Tocopherol selbst. Weiterhin betrifft die Erfindung neue Zwischenprodukte in diesem Verfahren.

Es sind bereits einige Verfahren zur Herstellung von natürlichem Vitamin E bekannt, welche jedoch technisch nur von begrenztem Interesse sind, so dass natürliches Vitamin E bis heute nahezu ausschliesslich aus natürlichen Quellen extrahiert wird.

Es bestand somit ein Bedürfnis nach einem technisch realisierbaren Verfahren, gemäss welchem natürliches Vitamin E in guter Ausbeute und mit hoher optischer Reinheit erhalten werden kann. Dies ist nunmehr mittels des erfindungsgemässen Verfahrens möglich.

Dieses Verfahren ist dadurch gekennzeichnet, dass man eine Verbindung der allgemeinen Formel

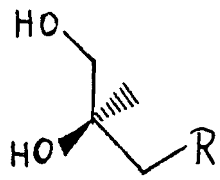

I

worin R eine Abgangsgruppe darstellt,
mit einer metallorganischen Verbindung der allgemeinen Formel

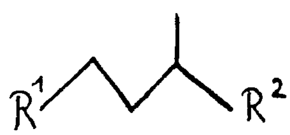

II

worin $R^1$ ein gegebenenfalls substituiertes Metallatom darstellt und $R^2$ einen Rest der Formel

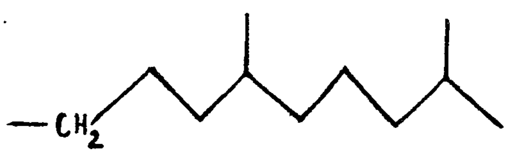.

$-CH(OR^3)_2$ oder $-CH_2R^4$
bedeutet, worin wiederum $R^3$ niederes Alkyl und $R^4$ Halogen oder eine Aethergruppe darstellen, umsetzt, dass man, gewünschtenfalls, eine so erhaltene Verbindung der Formel

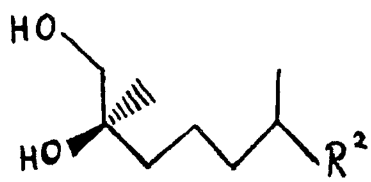

III

worin $R^2$ die obige Bedeutung hat,
in eine Verbindung der allgemeinen Formel

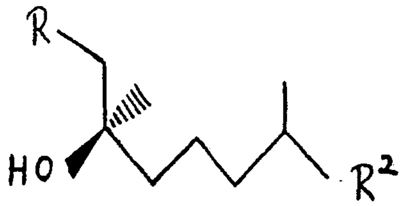

IV

worin R und $R^2$ die obige Bedeutung haben,
überführt, dass man, gewünschtenfalls, eine so erhaltene Verbindung der allgemeinen Formel IV in das Epoxid der Formel

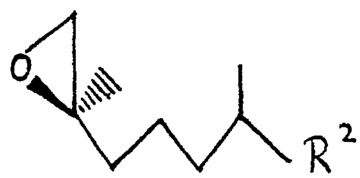

V

worin $R^2$ die obige Bedeutung hat,
überführt, dass man, gewünschtenfalls, das Epoxid der Formel V mit einer Verbindung der allgemeinen Formel

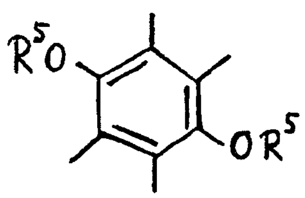

VI

worin $R^5$ eine Aetherschutzgruppe oder ein Aequivalent eines Alkalimetalls oder Erdalkalimetalls darstellt, umsetzt und dass man, gewünschtenfalls, eine so erhaltene Verbindung der allgemeinen Formel

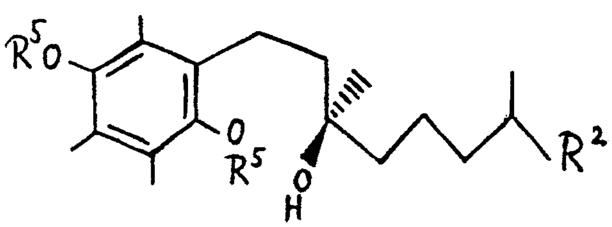

VII

worin $R^5$ die obige Bedeutung hat,
in d-α-Tocopherol überführt.

Der Ausdruck "Abgangsgruppe" bedeutet im Rahmen der vorliegenden Erfindung sowohl Halogen wie Chlor, Brom oder Jod, als auch Sulfonsäureester wie Tosylat oder Mesylat oder auch Carbonsäureester wie Acetat, Benzoat und dergleichen.

Als Metallatome kommen im Rahmen der vorliegenden Erfindung die für metallorganische Kopplungsreaktionen üblicherweise verwendeten Metalle in Frage, wie etwa Alkali- oder Erdalkalimetalle wie Lithium, Natrium, Kalium, Magnesium, oder auch Uebergangsmetalle wie Kupfer, Titan, Zink, Quecksilber und dergleichen, wobei Magnesium bevorzugt ist. Als Substituenten des Metallatoms kommen insbesondere in Frage Halogen wie Chlor, Brom oder Jod, oder auch niedere Alkoxygruppen mit 1 bis 6 Kohlenstoffatomen.

Der Ausdruck "niederes Alkyl" bedeutet im Rahmen der vorliegenden Erfindung Alkylgruppen mit 1 bis 6 Kohlenstoffatomen, welche geradkettig oder verzweigt sein können, wie etwa Methyl, Aethyl, Propyl, Butyl, Pentyl, Hexyl usw. Der Ausdruck "niederes Alkoxy" bedeutet Gruppen in denen der Alkylrest die

vorhergehende Bedeutung hat. Der Ausdruck "Halogen" bedeutet insbesondere Fluor, Chlor und Brom, wobei Chlor und Brom bevorzugt sind.

Der Ausdruck "Aetherschutzgruppe" bedeutet im Rahmen der vorliegenden Erfindung sowohl durch Hydrolyse spaltbare Gruppen wie etwa die Silylgruppe oder Alkoxymethylgruppen, beispielsweise die Methoxymethylgruppe, oder auch die Tetrahydropyranylgruppe, als auch oxidativ spaltbare Gruppen wie etwa $C_1$-$C_6$-Alkyläthergruppen. Der Schutz der phenolischen Hydroxylgruppen kann aber andererseits auch durch eine entsprechende Metallsalzgruppierung, nämlich durch ein Alkali- oder Erdalkalimetallsalz VI erzielt werden. Bevorzugt sind Natrium, Lithium, Kalium.

Weiterhin bedeutet das Zeichen

" 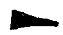 ",

dass sich der entsprechende Rest oberhalb der Molekülebene befindet, während das Zeichen

" 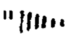 "

bedeutet, dass sich der entsprechende Rest unterhalb der Molekülebene befindet.

Die Umsetzung einer Verbindung der allgemeinen Formel I mit einer Verbindung der allgemeinen Formel II erfolgt zweckmässig dergestalt, dass man zunächst eine Verbindung der Formel I in einem inerten organischen Lösungsmittel mit einem Alkalimetall- oder Erdalkalimetallhydrid bis zum Abklingen der Wasserstoffentwicklung reagieren lässt und anschliessend eine Verbindung der Formel II zusetzt.

Als Alkali- oder Erdalkalihydride können im Rahmen der vorliegenden Erfindung insbesondere verwendet werden Lithium-, Natrium- oder Kaliumhydrid oder Calciumhydrid. Die Temperatur und der Druck sind bei dieser Reaktion keine kritischen Grössen. Bevorzugt erfolgt die Reaktion bei Raumtemperatur und Normaldruck.

Die Ueberführung einer Verbindung der allgemeinen Formel III in eine Verbindung der allgemeinen Formel IV kann in an sich bekannter Weise erfolgen. Zweckmässig erfolgt dies durch Umsetzung mit Säurehalogeniden oder Imidazoliden in inerten organischen Lösungsmitteln, vorzugsweise unter Zusatz einer organischen Base wie tert. Amine, z.B. Pyridin, Triäthylamin usw.

Die Ueberführung einer Verbindung der allgemeinen Formel IV in ein Epoxyd der allgemeinen Formel V kann in an sich bekannter Weise, d.h. durch Behandlung mit einer Base, erfolgen. Als Basen eignen sich sowohl anorganische als auch organische Basen, vorzugsweise jedoch anorganische Basen wie insbesondere Natriumhydroxid oder Kaliumhydroxid oder auch Natriumhydrid oder Calciumhydrid und dergleichen.

Die Umsetzung eines Epoxids der allgemeinen Formel V mit einer Verbindung der allgemeinen Formel VI erfolgt zweckmässig dergestalt, dass man entweder eine Verbindung der Formel VI, nach Zugabe einer geeigneten Alkalialkyl- oder Erdalkalialkylverbindung, in einem inerten organischen Lösungsmittel mit dem Epoxid reagieren lässt, oder dass man eine Verbindung der allgemeinen Formel VI in einem inerten organischen Lösungsmittel mit dem Epoxid in Gegenwart eines Alkali- oder Erdalkalimetallhydrids und eines Phasentransferkatalysators erhitzt, zweckmässig auf eine Temperatur von etwa 60 °C bis etwa Rückflusstemperatur des Reaktionsgemisches und gegebenenfalls unter Druck.

Als inerte organische Lösungsmittel können, im Rahmen der vorliegenden Erfindung, die üblicherweise bei metallorganischen Reaktionen verwendeten Lösungsmittel verwendet werden. Als Beispiele können hier genannt werden Aether, insbesondere cyclische Aether wie Tetrahydrofuran oder Dioxan oder auch Gemische dieser Aether mit aliphatischen Kohlenwasserstoffen wie insbesondere Pentan, Hexan und dergleichen.

Die im Rahmen der vorliegenden Erfindung benötigten Phasentransferkatalysatoren sind die üblicherweise verwendeten, bekannten Phasentransferkatalysatoren wie z.B. quaternäre Ammoniumsalze, Kronenäther oder auch Polyäther und dergleichen. Als Alkalialkyl- oder Erdalkalialkylverbindungen kommen im Rahmen der vorliegenden Erfindung z.B. in Frage tert.Butyllithium, $C_1$-$C_6$-Alkylkalium, $C_1$-$C_6$-Alkylnatrium, Grignard-Verbindungen und dergleichen.

Die nach dem erfindungsgemässen Verfahren erhaltenen Verbindungen der allgemeinen Formel VII, worin $R^2$ den Rest der Formel

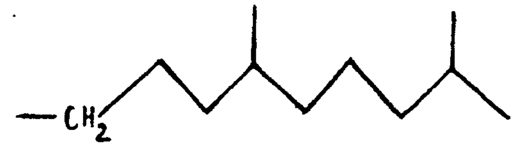

darstellt, sind z.B. aus EP-A-0 107 806 und EP-A-0 129 252 bekannt und können in bekannter Weise in d-α-Tocopherol übergeführt werden.

Diejenigen Verbindungen der Formel VII, worin $R^2$ einen Rest der Formel $-CH(OR^3)_2$ oder $-CH_2R^4$ bedeutet, sind z.B. aus hingegen neu und ebenfalls Gegenstand der vorliegenden Erfindung. Die Ueberführung einer derartigen Verbindung in eine Verbindung der Formel VII, worin $R^2$ den Rest

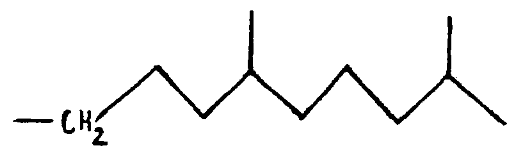

darstellt, kann in an sich bekannter Weise erfolgen, z.B. durch Umsetzung mit einem Phosphoniumsalz der Formel

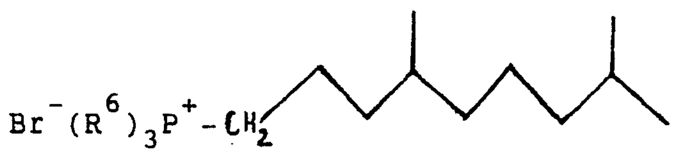,

worin $R^6$ einen Phenylrest darstellt, unter den für Wittig-Reaktionen oder auch anderen metallorganischen Reaktionen üblichen Bedingungen.

Anderseits kann in einer Verbindung der Formel VII, worin $R^2$ einen Rest der Formel $-CH(OR^3)_2$ oder $-CH_2R^4$ darstellt, zunächst der Chromanring in an sich bekannter Weise geschlossen werden, z.B. durch saure Hydrolyse oder durch Oxidation und anschliessende reduktive Cylisierung. Hierauf kann in Analogie zum Vorhergehenden in einer so erhaltenen Verbindung die Seitenkette verlängert werden.

Die in dem erfindungsgemässen Verfahren als Ausgangsmaterial verwendeten Verbindungen der Formeln I und II sind bekannte Verbindungen oder Analoge bekannter Verbindungen, welche in zur Herstellung der bekannten Verbindungen analoger Weise hergestellt werden können.

Die nachfolgenden Beispiele illustrieren die Erfindung und stellen keinerlei Einschränkung hiervon dar.

Beispiel 1

367 mg (3 mMol) (2S)-3-Chlor-2-methyl-1,2-propandiol wurden in 5 ml trockenem Tetrahydrofuran gelöst und mit 100 mg (7,2 mMol) Natriumhydrid (55-60%) bei Raumtemperatur bis zur abgeklungenen Gasentwicklung reagieren gelassen. Zur entstandenen dicken Suspension wurden 350 mg Kalium-tert.-butylat zugegeben. Hierauf wurden 2,9 mMol (3RS,7RS)-3,7,11-Trimethyldodecylmagnesiumbromid zugegeben und das Gemisch während 16 Stunden bei Raumtemperatur gerührt. Anschliessend wurden 50 ml Wasser zugegeben und das ganze mit Aether extrahiert. Die organischen Phasen wurden über Natriumsulfat getrocknet und eingeengt. Man erhielt 0,81 g (96%) (2R,6RS,10RS)-2,6,10,14-Tetramethyl-1,2-pentadecandiol mit $[\alpha]_D^{20}$ = +1,35° (c=1,5% in $CHCl_3$).

Das als Ausgangsmaterial verwendete (2S)-2-Methyl-3-chlorpropan-1,2-diol wurde wie folgt hergestellt:

7,9 ml (26 mMol) Tetraisopropyl-ortho-titanat, 150 mg (2,5 mMol) Calciumhydrid, 150 mg (2,5 mMol) Kieselgel und 5 ml (30 mMol) Dibutyl-L-tartrat wurden 10 Minuten bei -18° C in 150 ml Methylenchlorid stehen gelassen. Dann wurden 1,6 ml (25 mMol) beta-Methallylalkohol und 7 ml (50 mMol) Cumolhydroperoxid (66% in Cumol) zugetropft und das Gemisch 16 Stunden bei -18° C stehen gelassen. Hierauf wurden 300 ml Aethyläther und 50 ml (0,35 Mol) Natronlauge (28%ig) zugegeben und 1,5 Stunden bei Raumtemperatur gerührt. Dann wurde mit Aether extrahiert, die organischen Phasen mit 20,3 g (0,1 Mol) Magnesiumchlorid versetzt und 16 Stunden bei Raumtemperatur gerührt. Anschliessend wurde filtriert, das Filtrat eingeengt und mit Wasserdampf Cumol und Cumolalkohol abdestilliert. Der Rückstand wurde eingeengt und man erhielt 2,47 g (2S)-3-Chlor-2-methyl-1,2-propandiol als farbloses Oel mit $[\alpha]_D^{20}$ = +5,4° (c= 3% in

$CHCl_3$) und einer optischen Reinheit von über 98% (e.e.) nach gaschromatographischer Methode (Mosher-Derivat).

Beispiel 2

0,81 g (2,8 mMol) (2R,6RS,10RS)-2,6,10,14-Tetramethyl-1,2-pentadecandiol und 540 mg (2,9 mMol) Tosylchlorid wurden während 16 Stunden bei Raumtemperatur in 2 ml Pyridin reagieren gelassen. Das Gemisch wurde dann mit 50 ml 1N Salzsäure versetzt und dreimal mit je 50 ml Diäthyläther extrahiert. Die organischen Phasen wurden über Natriumsulfat getrocknet und eingeengt. Man erhielt 1,22 g (99%) (2R,6RS,10RS)-1-Tosyloxy-2,6,10,14-tetramethyl-3-pentadecanol mit $[\alpha]_D^{20}$ = -1,48° (c=2% in $CHCl_3$).

Beispiel 3

760 mg (2,9 mMol) 2,5-Di-(methoxymethoxy)-1,3,4,6-tetramethylbenzol wurden in 20 ml Tetrahydrofuran gelöst, mit 330 mg Kalium-tert.butylat und anschliessend mit 2 ml (3,2 mMol) n-Butyllithium bei Raumtemperatur versetzt und 1 Stunde reagieren lassen. Das Gemisch wurde dann mit (2R,6RS,10RS)-1,2-Epoxy-2,6,10,14-tetramethyl-pentadecan, hergestellt aus 1,22 g (2R,6RS,10RS)-1-Tosyloxy-2,6,10,14-tetramethyl-3-pentadecanol und 500 mg (3,1 mMol) Natriumhydrid in 30 ml Tetrahydrofuran, 16 Stunden bei Raumtemperatur reagieren gelassen. Hierauf wurden 10 ml HBr (30%ig) in 50 ml Methanol zugesetzt und das Gemisch 24 Stunden stehen gelassen. Anschliessend wurde Methanol zugesetzt und dann zur Trockene eingeengt. Der Rückstand wurde mit Toluol über Kieselgel filtriert und man erhielt 1,1 g (88%) (2R,4'RS,8'RS)-α-Tocopherol.

Beispiel 4

1,52 g (3,3 mMol) (2R,6RS,10RS)-2,6,10,14-Tetramethyl-1-tosyloxy-2-pentadecanol wurden in 20 ml Aethanol gelöst. Dann wurden 2 ml Natronlauge (50%ig) zugesetzt und das Gemisch 16 Stunden gerührt. Anschliessend wurden 50 ml Wasser zugegeben und das Ganze mit HYFLO verrührt. Die Suspension wurde dann filtriert und mit Wasser gewaschen. Der Rückstand wurde mit Diäthyläther verrührt, über Natriumsulfat getrocknet, filtriert und eingeengt. Man erhielt 0,63 g (2R,6RS,10RS)-1,2-Epoxy-2,6,10,14-tetramethylpentadecan mit $[\alpha]_D^{20}$ = -0,21° (c=2% in $CHCl_3$).

Beispiel 5

1,8 g (9 mMol) 2,5-Dimethoxy-1,3,4,6-tetramethylbenzol wurden in 100 ml Tetrahydrofuran gelöst, mit 1 g (9 mMol) Kalium-tert.butylat versetzt und anschliessend wurden 6 ml (9,5 mMol) n-Butyllithium (1,6 molar) bei Raumtemperatur zugetropft. Danach wurde das Gemisch 30 Minuten bei Raumtemperatur gerührt, tropfenweise mit 2,7 g (9 mMol) (2RS,6RS,10RS)-1,2-Epoxy-2,6,10,14-tetramethylpentadecan versetzt und 16 Stunden bei Raumtemperatur gerührt. Hierauf wurden HYFLO und 200 ml Wasser zugegeben, dann das Gemisch filtriert und mit Wasser gewaschen. Der Rückstand wurde mit Diäthyläther und Natriumsulfat verrührt, filtriert, mit Diäthyläther gewaschen und eingeengt. Man erhielt 3,8 g (3RS,7RS,11RS)-1-(2,5-Dimethoxy-3,4,6-trimethylphenyl)-3,7,11,15-tetramethyl-3-hexadecanol.

Beispiel 6

10 g (66,7 mMol) Tetramethylhydrochinon werden in 100 ml trockenem Tetrahydrofuran gelöst; es werden 4 g (139 mMol) Natriumhydrid (80% in Mineralöl) bei Raumtemperatur zugegeben, anschliessend 45 ml Butyllithium (1,6 molar in Hexan) zugetropft und es wird 1 Stunde nachgerührt. Es wurden nun 10 g (44,3 mMol) 1,2-Epoxi-2,6,10,14-tetramethylpentadecan zugetropft; unter Rühren wird 12 Stunden reagieren gelassen. Es erfolgt Zugabe von 100 ml 1N methanolischer Salzsäure und es wird 2 Stunden bei 50° gerührt. Anschliessend wird bei 50° eingeengt, und der Rückstand wird mit Toluol an Kieselgel chromatographiert, wobei 14,9 g (79%) α-Tocopherol erhalten wurden.

**Patentansprüche**

1. Verfahren zur Herstellung von tertiären Methylcarbinolderivaten bzw. von α-Tocopherol, dadurch gekennzeichnet, dass man eine Verbindung der allgemeinen Formel

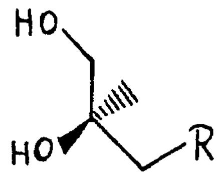

I

worin R eine Abgangsgruppe darstellt, mit einer metallorganischen Verbindung der allgemeinen Formel

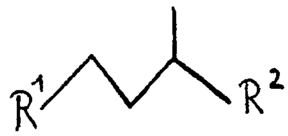

II

worin $R^1$ ein gegebenenfalls substituiertes Metallatom darstellt und $R^2$ einen Rest der Formel

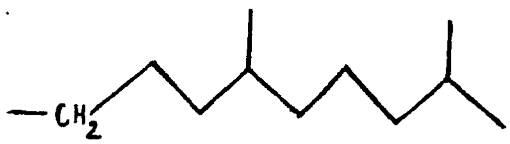

,

$-CH(OR^3)_2$ oder $-CH_2R^4$

bedeutet, worin wiederum $R^3$ $C_{1-6}$- Alkyl und $R^4$ Halogen oder eine Aethergruppe darstellen, umsetzt, dass man, gewünschtenfalls, eine so erhaltene Verbindung der Formel

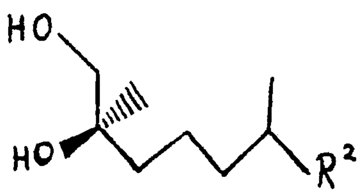

III

worin $R^2$ die obige Bedeutung hat, in eine Verbindung der allgemeinen Formel

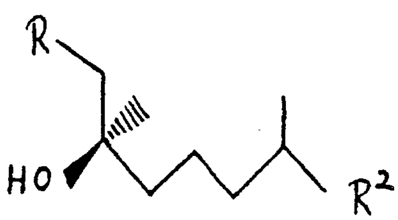

IV

worin R und $R^2$ die obige Bedeutung haben, überführt, dass man, gewünschtenfalls, eine so erhaltene Verbindung der Formel IV in das Epoxid der Formel

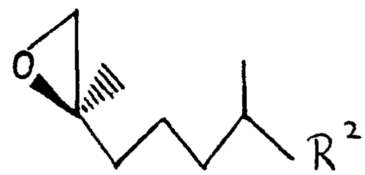

V

worin $R^2$ die obige Bedeutung hat, überführt, dass man, gewünschtenfalls, das Epoxid der Formel V mit einer Verbindung der allgemeinen Formel

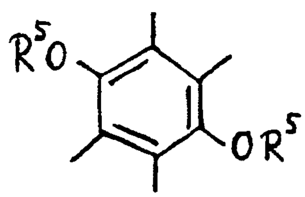

VI

worin $R^5$ eine Aetherschutzgruppe oder ein Aequivalent eines Alkalimetalls oder Erdalkalimetalls darstellt, umsetzt und, gewünschtenfalls, eine so erhaltene Verbindung der allgemeinen Formel

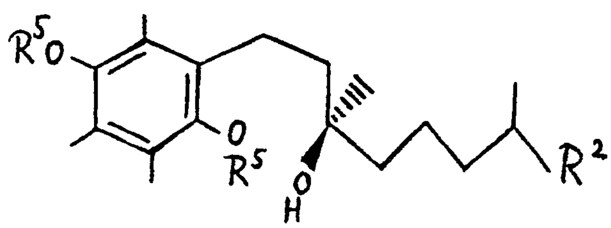

VII

worin $R^5$ obige Bedeutung hat, in d-α-Tocopherol überführt.

2. Verfahren gemäss Anspruch 1, dadurch gekennzeichnet, dass man eine Verbindung der allgemeinen Formel I in einem inerten organischen Lösungsmittel mit einem Alkalimetall-oder Erdalkalimetallhydrid bis zum Abklingen der Wasserstoffentwicklung reagieren lässt und anschliessend eine Verbindung der allgemeinen Formel II zusetzt.

3. Verfahren nach Anspruch 1 oder 2, dadurch gekennzeichnet, dass $R^5$ in der Verbindung VI eine Aetherschutzgruppe darstellt.

4. Verbindungen der allgemeinen Formel

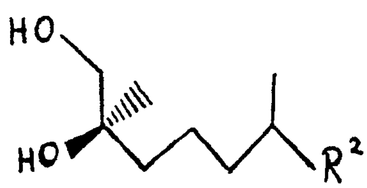

III

worin $R^2$ einen Rest der Formel

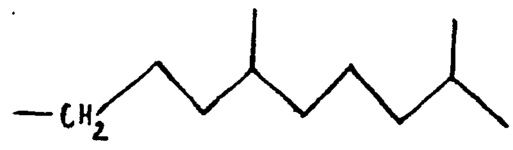,

$-CH(OR^3)_2$ oder $-CH_2R^4$
bedeutet, worin wiederum $R^3$ $C_{1-6}$- Alkyl und $R^4$ Halogen oder eine Aethergruppe darstellen.

**5.** Verbindungen der allgemeinen Formel

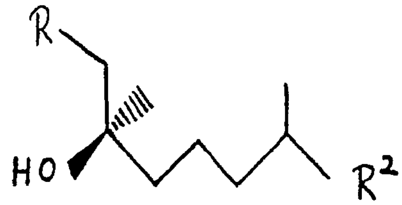

IV

worin R eine Abgangsgruppe und $R^2$ einen Rest der Formel

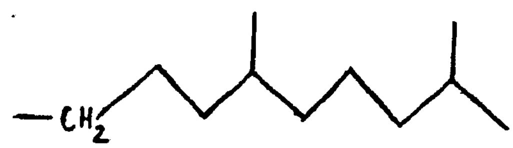,

$-CH(OR^3)_2$ oder $-CH_2R^4$
bedeutet, worin wiederum $R^3$ $C_{1-6}$- Alkyl und $R^4$ Halogen oder eine Aethergruppe darstellen.

**6.** Verbindungen der allgemeinen Formel

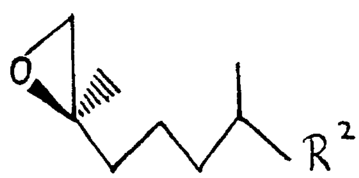

V

worin $R^2$ einen Rest der Formel

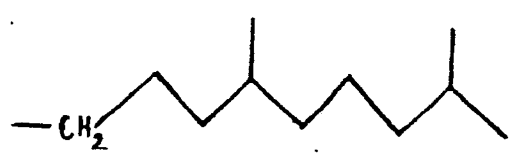,

$-CH(OR^3)_2$ oder $-CH_2R^4$
bedeutet, worin wiederum $R^3$ $C_{1-6}$- Alkyl und $R^4$ Halogen oder eine Aethergruppe darstellen.

**7.** Verbindungen der allgemeinen Formel

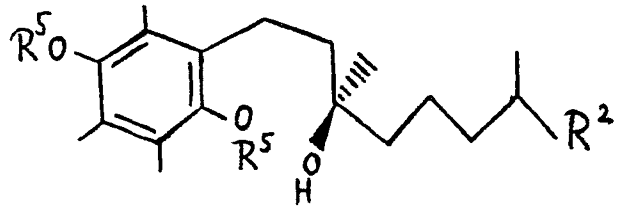

VII

worin $R^2$ einen Rest der Formel $-CH(OR^3)_2$ oder $-CH_2R^4$ darstellt, worin $R^3$ $C_{1-6}$- Alkyl und $R^4$ Halogen oder eine Aethergruppe darstellen und $R^5$ eine Aetherschutzgruppe oder ein Aequivalent eines Alkalimetalls oder Erdalkalimetalls bedeutet.

**8.** Verbindungen der allgemeinen Formel VII gemäss Anspruch 7, dadurch gekennzeichnet, dass $R^5$ eine Aetherschutzgruppe bedeutet.

**Claims**

**1.** A process for the manufacture of tertiary methylcarbinol derivatives or of α-tocopherol, characterized by reacting a compound of the general formula

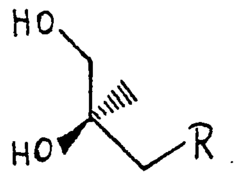

I

wherein R represents a leaving group,
with a metal-organic compound of the general formula

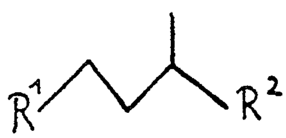

II

wherein $R^1$ represents an optionally substituted metal atom and $R^2$ signifies a residue of the formula

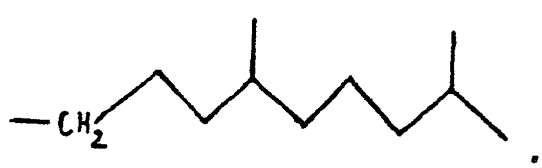

$-CH(OR^3)_2$ or $-CH_2R^4$
in which $R^3$ represents $C_{1-6}$-alkyl and $R^4$ represents halogen or an ether group,
and, if desired, converting a thus-obtained compound of the formula

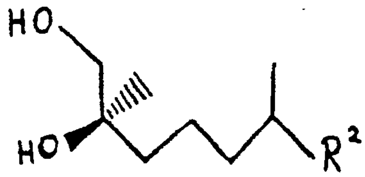

III

wherein $R^2$ has the above significance,
into a compound of the general formula

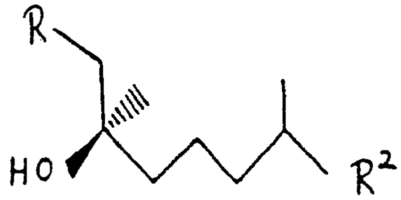

IV

wherein R and $R^2$ have the above significance,
if desired, converting a thus-obtained compound of general formula IV into an epoxide of the formula

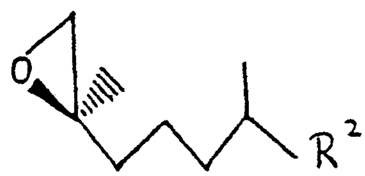

V

wherein $R^2$ has the above significance,
if desired, reacting an epoxide of formula V with a compound of the general formula

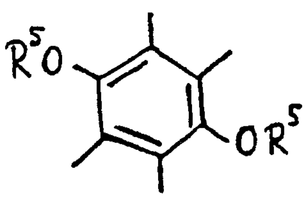

VI

wherein $R^5$ represents an ether protecting group or one equivalent of an alkali metal or alkaline earth metal,
and, if desired, converting a thus-obtained compound of the general formula

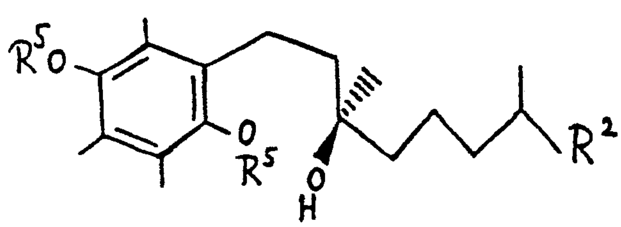

VII

wherein $R^5$ has the above significance,
into d-α-tocopherol.

2. A process in accordance with claim 1, characterized in that a compound of general formula I is reacted in an inert organic solvent with an alkali metal hydride or alkaline earth metal hydride until the hydrogen evolution has finished and subsequently a compound of general formula II is added.

3. A process in accordance with claim 1 or claim 2, characterized in that $R^5$ in the compound of formula VI represents an ether protecting group.

4. Compounds of the general formula

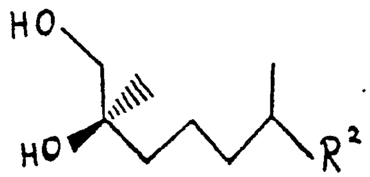

III

wherein $R^2$ signifies a residue of the formula

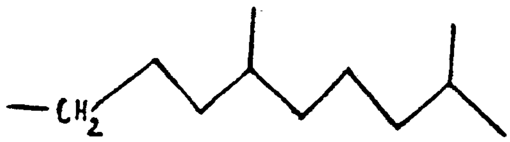

$-CH(OR^3)_2$ or $-CH_2R^4$
in which $R^3$ represents $C_{1-6}$-alkyl and $R^4$ represents halogen or an ether group.

5. Compounds of the general formula

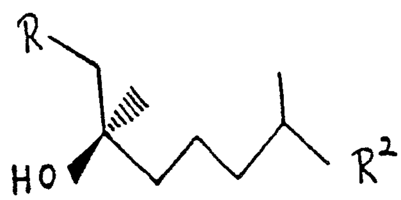

IV

wherein R signifies a leaving group and $R^2$ signifies a residue of the formula

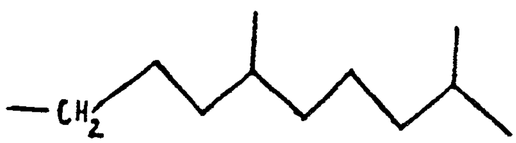

$-CH(OR^3)_2$ or $CH_2R^4$
in which $R^3$ represents $C_{1-6}$-alkyl and $R^4$ represents halogen or an ether group.

6. Compounds of the general formula

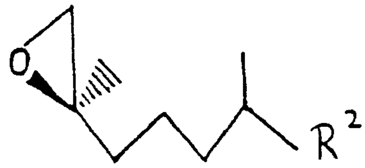

V

wherein $R^2$ signifies a residue of the formula

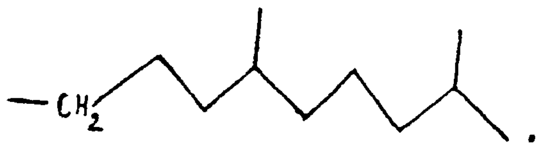

$-CH(OR^3)_2$ or $-CH_2R^4$
in which $R^3$ represents $C_{1-6}$-alkyl and $R^4$ represents halogen or an ether group.

7. Compounds of the general formula

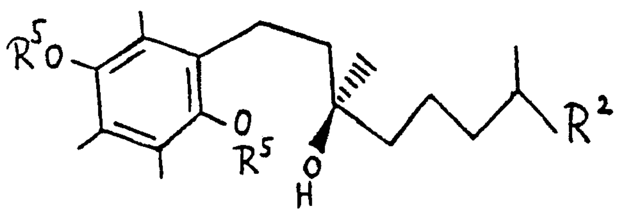

VII

wherein $R^2$ represents a residue of the formula $-CH(OR^3)_2$ or $-CH_2R^4$ in which $R^3$ represents $C_{1-6}$-alkyl and $R^4$ represents halogen or an ether group, and $R^5$ signifies an ether protecting group or one equivalent of an alkali metal or alkaline earth metal.

8. Compounds of general formula VII in accordance with claim 7, characterized in that $R^5$ represents an ether protecting group.

**Revendications**

1. Procédé de préparation de dérivés tertiaires du méthylcarbinol ou de l'alpha-tocophérol, caractérisé en ce que l'on fait réagir un composé de formule générale

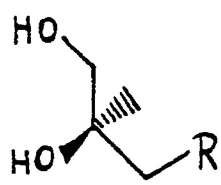

I

dans laquelle R représente un groupe éliminable, avec un composé organométallique de formule générale

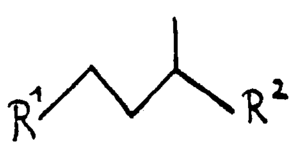

II

dans laquelle $R^1$ représente un atome métallique éventuellement substitué et $R^2$ un groupe de formule

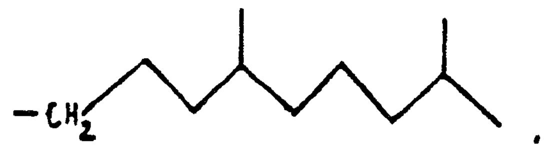

$-CH(OR^3)_2$ ou $-CH_2R^4$

dans lesquelles $R^3$ représente un groupe alkyle en C 1-C 6 et $R^4$ un halogène ou un groupe éther, et, si on le désire, on convertit un composé ainsi obtenu, répondant à la formule

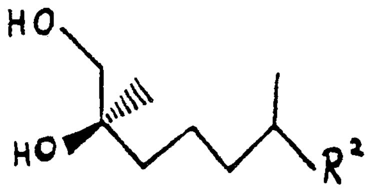

III

dans laquelle $R^2$ a les significations indiquées ci-dessus, en un composé de formule générale

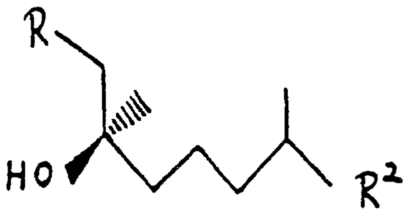

IV

dans laquelle R et $R^2$ ont les significations indiquées ci-dessus,

et, si on le désire, on convertit un composé ainsi obtenu, répondant à la formule IV, en l'époxyde de formule

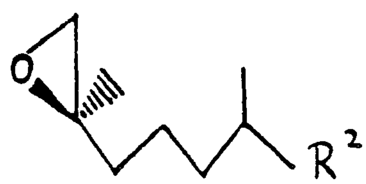

V

dans laquelle $R^2$ a les significations indiquées ci-dessus, après quoi, si on le désire, on fait réagir l'époxyde de formule V avec un composé de formule générale

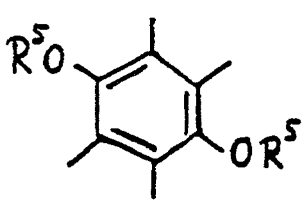

VI

dans laquelle $R^5$ représente un groupe protecteur éther ou un équivalent d'un métal alcalin ou alcalino-terreux, et, si on le désire, on convertit un composé ainsi obtenu, répondant à la formule générale

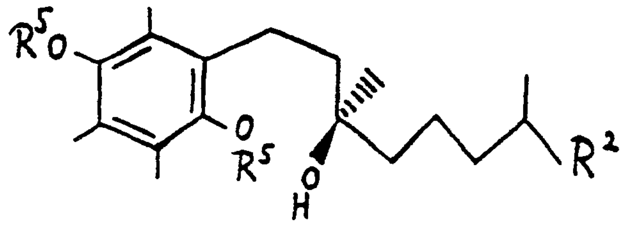

VII

dans laquelle $R^5$ a les significations indiquées ci-dessus, en le d-alpha-tocophérol.

**2.** Procédé selon la revendication 1, caractérisé en ce que l'on fait réagir un composé de formule générale I, dans un solvant organique inerte, avec un hydrure de métal alcalin ou alcalino-terreux, jusqu'à cessation du dégagement d'hydrogène, puis on ajoute un composé de formule générale II.

**3.** Procédé selon la revendication 1 ou 2, caractérisé en ce que le symbole $R^5$, pour le composé VI, représente un groupe protecteur éther.

**4.** Composés de formule générale

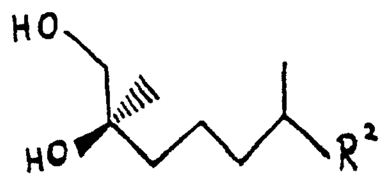

III ,

dans laquelle $R^2$ représente un groupe de formule

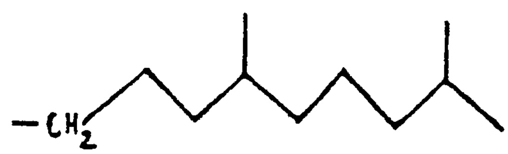

.

$-CH(OR^3)_2$ ou $-CH_2R^4$
dans lesquelles $R^3$ représente un groupe alkyle en C 1-C 6 et $R^4$ un halogène ou un groupe éther.

**5.** Composés de formule générale

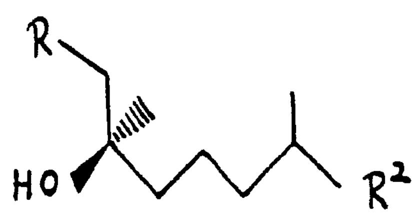

IV

dans laquelle R représente un groupe éliminable et $R^2$ un groupe de formule

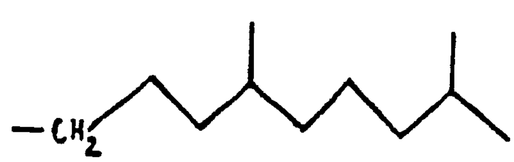

.

$-CH(OR^3)_2$ ou $-CH_2R^4$
dans lesquelles $R^3$ représente un groupe alkyle en C 1-C 6 et $R^4$ un halogène ou un groupe éther.

**6.** composés de formule générale

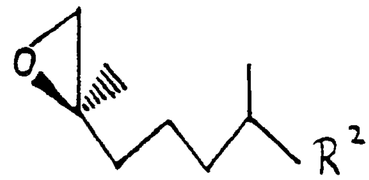

V

dans laquelle $R^2$ représente un groupe de formule

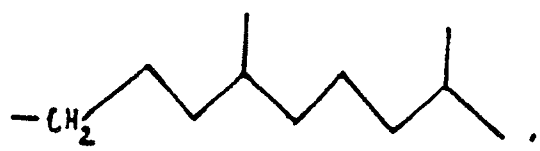

$-CH(OR^3)_2$ ou $-CH_2R^4$
dans lesquelles $R^3$ représente un groupe alkyle en C 1-C 6 et $R^4$ un halogène ou un groupe éther.

**7.** composés de formule générale

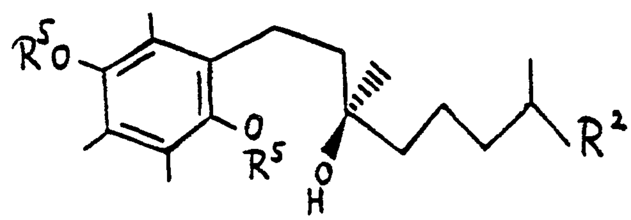

VII

dans laquelle $R^2$ représente un groupe de formule $-CH(OR^3)_2$ ou $-CH_2R^4$ dans lesquelles $R^3$ représente un groupe alkyle en C 1-C 6 et $R^4$ un halogène ou un groupe éther et $R^5$ représente un groupe protecteur éther ou un équivalent d'un métal alcalin ou alcalino-terreux.

**8.** composés de formule générale VII selon la revendication 7, caractérisés en ce que $R^5$ représente un groupe protecteur éther.